# EUROPEAN PATENT SPECIFICATION

(11) **EP 4 202 845 B1**
(45) Date of publication and mention of the grant of the patent: **01.04.2026**
(21) Application number: 21857776.5
(22) Date of filing: 20.08.2021
(51) Int. Cl.: G01B 11/25, G06T 17/00, A61B 5/00, A61C 9/00, A61C 19/04, G06T 7/521, H04N 13/254, G06T 7/55, G06T 5/50

(54) **DATA RECONSTRUCTION METHOD AND SYSTEM, AND SCANNING DEVICE**
DATENREKONSTRUKTIONSVERFAHREN UND -SYSTEM SOWIE ABTASTVORRICHTUNG
PROCÉDÉ ET SYSTÈME DE RECONSTRUCTION DE DONNÉES, ET DISPOSITIF DE BALAYAGE

(30) Priority: 21.08.2020 CN 202010851553
(43) Date of publication of application: 28.06.2023
(73) Proprietor: Shining 3D Tech Co., Ltd., Hangzhou, Zhejiang 311258 (CN)
(72) Inventor: MA, Chao, Hangzhou, Zhejiang 311258 (CN)
(74) Representative: Novagraaf Group
(86) International application number: PCT/CN2021/113854
(87) International publication number: WO 2022/037688

(56) References cited:
- EP-A1- 3 064 894
- CN-A- 101 694 375
- CN-A- 101 694 375
- CN-A- 109 584 352
- CN-A- 109 916 336
- CN-A- 110 619 601
- CN-A- 110 702 031
- CN-U- 201 548 210
- US-A1- 2019 282 342
- US-B2- 8 786 679
- WANG JIANHUA ET AL: "Three-Dimensional Shape Detection for Non Uniform Reflective Objects: Combination of Color Light Projection and Camera's Exposure Adjustment", IEEE SENSORS JOURNAL, IEEE, USA, vol. 20, no. 19, 20 May 2020 (2020-05-20), pages 11474 - 11483, XP011807423, ISSN: 1530-437X, [retrieved on 20200902], DOI: 10.1109/JSEN.2020.2996510
- HONGZHI JIANG ET AL: "High dynamic range fringe acquisition: A novel 3-D scanning technique for high-reflective surfaces", OPTICS AND LASERS IN ENGINEERING, ELSEVIER, AMSTERDAM, NL, vol. 50, no. 10, 10 November 2011 (2011-11-10), pages 1484 - 1493, XP028500063, ISSN: 0143-8166, [retrieved on 20120511], DOI: 10.1016/J.OPTLASENG.2011.11.021

## Description

The present application claims the benefit of priority of Chinese patent application No.202010851553.3, entitled "METHOD AND SYSTEM FOR RECONSTRUCTING DATA AND SCANNING DEVICE", filed to China National Intellectual Property Administration on August 21, 2020.

### Technical Field

The present disclosure relates to the technical field of data processing, and more particularly relates to a method and system for reconstructing data and a scanning device.

### Background

In related art, a means for acquiring dental cast data turns into an intraoral three-dimensional scanning technology from impression three-dimensional scanning. According to the intraoral three-dimensional scanning technology, an intraoral three-dimensional scanner directly goes deep into a mouth to acquire teeth three-dimensional data by scanning, but due to more specular reflection of metal materials, the intraoral three-dimensional scanner usually adopts a multi-angle and multi-time scanning manner when acquiring data of this kind of materials, which reduces data acquiring efficiency and makes a scanning operator and a patient uncomfortable. Particularly, when there is an optical dead angle, the intraoral three-dimensional scanner cannot acquire complete data, which increases a repair workload for later design and denture wearing.

US 2019/282342 A1 relates to an intraoral scanner comprising a handle, a mouthpiece extending from the handle, a structured light projector projecting a light pattern from the mouthpiece, and a stereo camera capturing images through the mouthpiece.

Wang Jianhua et al deals with "Three-Dimensional Shape Detection for Non Uniform Reflective Objects: Combination of Color Light Projection and Camera's Exposure Adjustment", IEEE SENSORS JOURNAL, IEEE, USA, vol. 20, no. 19, pages 11474 - 11483. 1

Hongzhi Jiang et al deals with "High dynamic range fringe acquisition: A novel 3-D scanning technique for high-reflective surfaces", OPTICS AND LASERS IN ENGINEERING, ELSEVIER, AMSTERDAM, NL, vol. 50, no. 10, pages 1484 - 1493.

CN 101 694 375 A relates to a stereoscopic vision detecting method for measuring three-dimensional morphology on strong reflection surface.

Meanwhile, high light reflection usually reduces an object imaging dynamic range, however, when the multi-angle and multi-time scanning manner is adopted for collecting intraoral tooth data, if there is a local area highly bright or very dark in a scanned object, a camera cannot acquire uniform-brightness images, which requires to utilize different perspectives for avoiding influences of light reflection on three-dimensional reconstruction. If an attempt is about to be made to change tooth optical characteristics, solid powder spraying or liquid coating is performed, in an existing method, on teeth and gums, powder or a coating is utilized for shielding incident light, and surface three-dimensional information of teeth underneath a powder layer can be restored by controlling the thickness of the powder or the coating. But the use of powder and coating is inconvenient due to the fact that, for example, the patient is allergic to or difficultly accepts the powder and the coating, which prolongs the whole scanning time; and the thickness of the powder or the coating directly influences precision of three-dimensional data, which covers defects of surfaces of teeth, and makes real tooth colors unavailable.

There are still no effective solutions for the above problems.

### Summary

The present disclosure provides a method and system for reconstructing data and a scanning device, so as to at least solve technical problems that in the related technologies, an intraoral scanner adopts a multi-angle and multi-time scanning manner to collect intraoral tooth data, and if there is a local area highly bright or very dark in a scanned object, a camera cannot acquire uniform-brightness images.

According to embodiments of the present disclosure, a method for reconstructing data is provided, including: collecting a plurality of sets of image sequences different in brightness level at the same position on a to-be-scanned object; and performing image fusion and three-dimensional reconstruction based on the plurality of sets of image sequences different in brightness level to generate three-dimensional data of the to-be-scanned object.

In some embodiments, the collecting a plurality of sets of image sequences different in brightness level at the same position on a to-be-scanned object includes: acquiring, by a three-dimensional scanner, the plurality of sets of image sequences at the same position based on different optical conditions.

In some embodiments, the method further includes: adjusting light source brightness and/or exposure parameters of a projection optical device so as to adjust optical conditions of the three-dimensional scanner, and/or adjusting exposure parameters and/or gain parameters of an image acquisition device so as to adjust optical conditions of the three-dimensional scanner.

In some embodiments, the step of collecting a plurality of sets of image sequences different in brightness level at the same position on a to-be-scanned object: namely, acquiring the plurality of sets of image sequences at the same position based on different optical conditions includes: collecting images of a first quantity set under a first optical condition, where the types of the images of the first quantity set include: code patterns, reconstruction patterns and texture patterns; collecting images of a second quantity set under a second optical condition, where the types of the images of the second quantity set include: reconstruction patterns and texture patterns; and/or, collecting images of a third quantity set under a third optical condition, where the types of the images of the third quantity set include: reconstruction patterns and texture patterns.

In some embodiments, the step of performing image fusion and three-dimensional reconstruction based on the plurality of sets of image sequences different in brightness level to generate three-dimensional data of the to-be-scanned object includes: performing fusion processing on the images of the first quantity set, the images of the second quantity set and the images of the third quantity set; performing three-dimensional reconstruction on the fused images to generate the three-dimensional data of the to-be-scanned object, where the three-dimensional data includes point cloud data and texture data; respectively performing three-dimensional reconstruction on the images of the first quantity set, the images of the second quantity set and the images of the third quantity set; and fusing reconstruction results of three-dimensional reconstruction to generate the three-dimensional data of the to-be-scanned object, where the three-dimensional data includes point cloud data and texture data.

According to the invention, before the collecting a plurality of sets of image sequences different in brightness level at the same position on a to-be-scanned object, the method further includes: acquiring images on a surface of the to-be-scanned object and assessing image uniformity; starting a first scanning mode if the images are uniform; and starting a second scanning mode if the images are not uniform, where the second scanning mode is a mode in which the plurality of sets of image sequences different in brightness level at the same position on the to-be-scanned object are collected and then subjected to fusion and three-dimensional reconstruction. In some embodiments, before the collecting a plurality of sets of image sequences different in brightness level at the same position on a to-be-scanned object, the method further includes: determining the brightness level of the image sequences according to the non-uniformity degree of the images.

In some embodiments, in the first scanning mode, image sequences are collected based on a preset fourth optical condition, where the image sequences include code patterns, reconstruction patterns and texture patterns; and three-dimensional reconstruction is performed based on the code patterns and the reconstruction patterns to obtain point cloud images, and texture images are obtained based on the texture patterns, where the texture images correspond to the point cloud images.

According to embodiments of the present disclosure, a system for reconstructing data is further provided, including: a projection optical device for adjusting optical conditions according to presetting so as to adjust light source brightness projected to a to-be-scanned object; an image acquisition device, configured to collect a plurality of sets of image sequences different in brightness level at the same position on the to-be-scanned object, where the different brightness levels are resulted from adjustment on the light source brightness by the projection optical device; and a processor configured to respectively communicate with the projection optical device and the image acquisition device and configured to perform image fusion and three-dimensional reconstruction based on the plurality of sets of image sequences different in brightness level to generate three-dimensional data of the to-be-scanned object.

According to embodiments of the present disclosure, a scanning device is further provided and includes a processor and a memory which is configured to store executable instructions of the processor, where the processor is configured to execute the executable instructions to execute any above method for reconstructing data.

According to embodiments of the present disclosure, a computer-readable storage medium is further provided and includes stored computer programs, where the computer programs, when operating, control a device where the computer-readable storage medium is located to execute any above method for reconstructing data.

In the present disclosure, the plurality of sets of image sequences different in brightness level at the same position on the to-be-scanned object are collected, the projection optical device projects the plurality of sets of image sequences to a surface of a detected object, and each set of image sequence includes one or more images. The projection optical device performs adjustment according to presetting, so that the images in any set of image sequence and the images in the other sets of image sequences are consistent but only different in brightness level. In this embodiment, the projection optical device adjusts exposure time according to the presetting, that is, the projection optical device projects one set of image sequence based on a first exposure time, and projects one set of image sequence based on a second exposure time, the image acquisition device collects the image sequences on the surface of the to-be-scanned object and thus acquires the image sequences different in brightness level, and image fusion and three-dimensional reconstruction are performed based on the plurality of sets of image sequences different in brightness level to generate the three-dimensional data of the to-be-scanned object. In the present disclosure, if there is a local area highly bright or very dark in the scanning process, image fusion and three-dimensional reconstruction can be performed based on the plurality of sets of image sequences different in brightness level at the same position, so that high-quality three-dimensional data can be acquired from to-be-scanned objects with different bright-dark materials, thereby solving technical problems that in the related technologies, an intraoral scanner adopts a multi-angle and multi-time scanning manner to collect intraoral tooth data, and if there is a local area highly bright or very dark in the scanned object, the camera cannot acquire uniform-brightness images.

### Brief Description of the Drawings

Drawings illustrated herein are used for providing further understanding for the present disclosure and constitute a part of the present disclosure. Schematic embodiments of the present disclosure and explanations thereof are used for explaining the present disclosure, which do not constitute improper limits to the present disclosure. In the drawings:
FIG. 1 is a flowchart of an optional method for reconstructing data according to an embodiment of the present disclosure; and
FIG. 2 is a schematic diagram of an optional system for reconstructing data according to an embodiment of the present disclosure.

### Detailed Description

For the purpose of making those skilled in the art better understand schemes of the present disclosure, technical schemes in embodiments of the present disclosure are clearly and completely described in conjunction with drawings in the embodiments of the present disclosure as below, and obviously, the ones described herein are merely a part of the embodiments of the present disclosure and not all the embodiments. Based on the embodiments of the present disclosure, all other embodiments obtained by those of ordinary skill in the art without creative work fall within the scope of protection of the present disclosure.

It needs to be explained that terms such as "first" and "second" in Description and Claims of the present disclosure and the above drawings are used for distinguishing similar objects but not necessarily used for describing specific sequences or precedence orders. It should be understood that adopted data can be exchanged under a proper situation so as to implement the embodiments, described herein, of the present disclosure in sequence except the illustrated or described sequences. In addition, terms "include" and "have" and any transformations thereof are intended to cover non-exclusive inclusion, for example, a process, a method, a system, a product or a device including a series of steps or units is not limited to clearly-listed steps or units, while may include unclearly-listed other steps or units or other inherent steps or units of the process, the method, the product or the device.

To facilitate those skilled in the art to understand the present disclosure, part of terms or nouns involved in the embodiments of the present disclosure are explained:
An oral cavity digital impression instrument, also called an intraoral three-dimensional scanner, is a device which applies a probe type optical scanning head to directly scan an oral cavity of a patient and acquire three-dimensional shape and colorful texture information of surfaces of soft or hard tissues such as teeth, gums and a mucous membrane in the oral cavity. A projection optical device is utilized for projecting an active light pattern, and an image acquisition device acquires the pattern and performs three-dimensional reconstruction and splicing by algorithm processing. Of course, principles adopted by the oral cavity digital impression instrument are not limited, for example, principles of microscopy confocal imaging, etc. can be adopted for image processing.

The projection optical device adopts a Digital Light Procession (DLP) projection technology and uses a Digital Micromirror Device (DMD) as a key processing element to realize a digital optical processing process. Due to a small pixel size of the projection optical device, interference between adjacent stripe patterns on teeth can be reduced, and non-erasable mutual effects between stripes are avoided due to a high-precision stripe center line extraction algorithm. The technology greatly reduces influences of enamel of the teeth on light transmission and diffusion. Through cooperation with adjustment on an included angle between an optical axis of the image acquisition device and an optical axis of the projection optical device, a high light reflection property of the teeth or saliva is greatly reduced.

The intraoral three-dimensional scanner in the embodiment of the present disclosure integrates a first scanning mode and a second scanning mode, and in a process of directly acquiring three-dimensional data of the teeth and the gums, when there are high light reflective materials such as metal restored teeth, the second scanning mode is started. The second scanning mode is implemented in a manner that the advantages that a DLP projector can adjust projection brightness in real time, and the image acquisition device adjusts exposure parameters and gain parameters in real time are utilized, at a current scanned position, the DLP projector is configured to have at least more than two brightness levels in cooperation with proper camera exposure parameters and gain parameters, thereby acquiring multi-level images at the same position (including: bright-level images and dark-level images, the bright-dark levels of the DLP projector being adjusted according to material conditions of to-be-scanned objects, such as 2 levels, 3 levels, four 4 and 5 levels, namely, scanning being performed based on two or three or other kinds of optical conditions in the second scanning mode of the three-dimensional scanner); then, three-dimensional reconstruction is respectively performed on multi-level image sequences, three-dimensional data obtained after reconstruction of the multi-level image sequences is fused into more perfect three-dimensional data, or the multi-level image sequences are fused to acquire more perfect image sequences, and then perfect three-dimensional data is obtained through three-dimensional reconstruction. In this mode, the mode can be applied to the local highly-bright area by at least two sets of image sequences different in brightness level. For the highly-bright part, the first scanning mode is switched into the second scanning mode for scanning, which can meet real-time scanning requirements under the high light reflection situation. The first scanning mode is implemented in a manner that optical parameters of the DLP projector and the image acquisition device are kept unchangeable, only image sequences the same in brightness level are collected at the same position, which is an ordinary scanning mode applicable to scanning under conventional situations.

In this embodiment, the projection optical device is the DLP projector, and the image acquisition device is a camera.

According to the embodiment of the present disclosure, an embodiment of a method for reconstructing data is provided. It needs to be explained that steps shown in a flowchart of the drawings may be performed in a computer system with a set of computer executable instructions. In addition, although a logical sequence is shown in the flowchart, the illustrated or described steps may be performed in sequence different from the sequence herein under some situations.

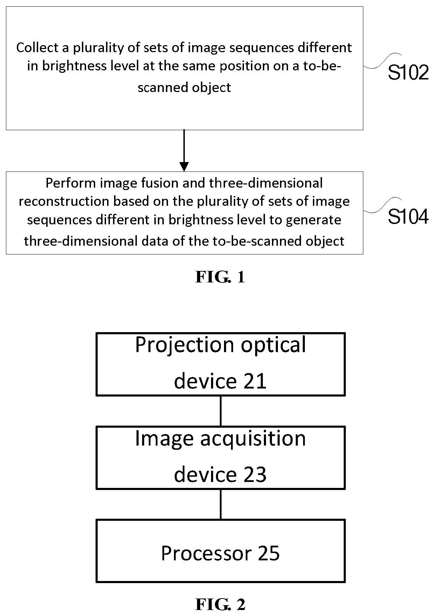

FIG. 1 is a flowchart of an optional method for reconstructing data according to an embodiment of the present disclosure. As shown in FIG. 1, the method includes following steps:
S102: A plurality of sets of image sequences different in brightness level at the same position on a to-be-scanned object are collected.

S104: Image fusion and three-dimensional reconstruction are performed based on the plurality of sets of image sequences different in brightness level to generate three-dimensional data of the to-be-scanned object.

Through the above steps, the plurality of sets of image sequences different in brightness level at the same position on the to-be-scanned object are collected, and the projection optical device projects the plurality of sets of image sequences to a surface of a detected object. Each set of image sequence includes one or more images. The projection optical device performs adjustment according to presetting, so that the images in any set of image sequence and the images in the other sets of image sequences are consistent but only different in brightness level. In this embodiment, the projection optical device adjusts exposure time according to the presetting, that is, the projection optical device projects one set of image sequence based on a first exposure time, and projects one set of image sequence based on a second exposure time, the image acquisition device collects the image sequences on the surface of the to-be-scanned object and thus acquires the image sequences different in brightness level, and image fusion and three-dimensional reconstruction are performed based on the plurality of sets of image sequences different in brightness level to generate the three-dimensional data of the to-be-scanned object. In this embodiment, if there is a local area highly bright or very dark in the scanning process, image fusion and three-dimensional reconstruction can be performed based on the plurality of sets of image sequences different in brightness level at the same position, so that high-quality three-dimensional data can be acquired from to-be-scanned objects with different bright-dark materials, thereby solving technical problems that in the related technologies, an intraoral scanner adopts a multi-angle and multi-time scanning manner to collect intraoral tooth data, and if there is a local area highly bright or very dark in the scanned object, the camera cannot acquire uniform-brightness images.

A scanning body of the embodiment of the present disclosure may be a scanning system, including but not limited to: the intraoral three-dimensional scanner and a computer. The intraoral three-dimensional scanner includes but not limited to: the DLP projector and a monocular black and white camera. Of course, there may be other combinations, such as a combination of the DLP projector, the monocular black and white camera and a texture camera or a combination of the DLP projector and the binocular black and white camera. The intraoral three-dimensional scanner integrates the first scanning mode and the second scanning mode, and the first scanning mode is switched into the second switching mode for scanning a highly-bright part. Of course, only the second scanning mode may be included, or other types of scanning modes may be included. The second scanning mode may also be subjected to mode subdivision, for example, the second scanning mode is subdivided into a second scanning mode A and a second scanning mode B, where the second scanning mode A includes a first brightness level, a second brightness level and a third brightness level; and the second scanning mode B includes a fourth brightness level and a fifth brightness level.

It needs to be explained that the scanning body of the embodiment of the present disclosure is not limited to an intraoral three-dimensional scanning system, and may also be a false tooth three-dimensional scanning system or other three-dimensional scanning systems.

The above steps are combined to describe the present disclosure in detail below.

S102: A plurality of sets of image sequences different in brightness level at the same position on a to-be-scanned object are collected.

Cases usually needing the intraoral three-dimensional scanner for scanning include: repair, orthodontics and implant. In the field of repair cases, the intraoral three-dimensional scanner usually meets false teeth previously repaired by the patient, such as metal teeth when acquiring tooth data. In the field of implant cases, the intraoral three-dimensional scanner needs to acquire data of intraoral teeth of the patient and meanwhile can directly scan a scanning rod, an abutment, etc. at a missing tooth position, and materials thereof include highly-reflective metal, bright-white materials, a titanium alloy, etc. The repaired metal teeth, the scanning rod, etc. all belong to highly-reflective objects, which bring big trouble to the intraoral three-dimensional scanner utilizing an optical imaging principle. Because specular reflection or light absorption occurs at different angles when light is projected onto such to-be-scanned objects, which will cause the image acquisition device to only acquire an overexposed image or too dark image and import a poor image quality for three-dimensional reconstruction. According to the embodiment of the present disclosure, the plurality of image sequences different in brightness level at the same position on the to-be-scanned object can be collected to acquire the high-quality three-dimensional data.

The projection optical device can adjust the brightness level of the projected light by adjusting the light source brightness (the light source brightness is adjusted commonly through a current value) or the exposure parameters, and the image acquisition device can adjust the brightness level of the acquired images by adjusting the exposure parameters or the gain parameters.

In some embodiments, the image acquisition device collects more sets of image sequences by adjusting the exposure parameters and the gain parameters in real time. In the embodiment of the present disclosure, the image acquisition device may be adjusted or may not be adjusted, which only needs to be matched with the plurality of brightness levels of the projection optical device to collect images different in brightness level. The exposure parameters include exposure time.

The intraoral three-dimensional scanner acquires the plurality of sets of image sequences at the same position based on different optical conditions, and the optical conditions of the intraoral three-dimensional scanner are adjusted by adjusting the light source brightness and/or the exposure parameters of the projection optical device, and/or by adjusting the exposure parameters and/or the gain parameters of the image acquisition device. Specifically, the intraoral three-dimensional scanner is configured to have at least more than two brightness levels to acquire the plurality of sets of image sequences different in brightness level at the same position, such as the bright-level images and the dark-level images. The bright-dark levels of the projection optical device are adjustable according to material conditions, such as 2 levels, 3 levels, 4 levels and 5 levels, so as to respectively perform three-dimensional reconstruction on the image sequences of the bright-dark levels, and more perfect three-dimensional data is obtained in combination with fusion of the plurality sets of three-dimensional data, or the image sequences of the bright-dark levels are fused to acquire more perfect image sequences, and then perfect three-dimensional data is obtained through three-dimensional reconstruction.

As an optional embodiment of the present disclosure, the step of collecting a plurality of sets of image sequences different in brightness level at the same position on a to-be-scanned object: namely, projecting, by the projection optical device, the image sequences to the surface of the detected object based on different exposure times and synchronously collecting, by the image acquisition device, the image sequences on the surface of the detected object to acquire the image sequences different in brightness level includes: the projection optical device projects images of a first quantity set to the surface of the detected object based on a first exposure time, and the image acquisition device synchronously collects the images of the first quantity set on the surface of the detected object, where the images of the first quantity set include code patterns, reconstruction patterns and texture patterns; the projection optical device projects images of a second quantity set to the surface of the detected object based on a second exposure time, and the image acquisition device synchronously collects the images of the second quantity set on the surface of the detected object, where the images of the second quantity set include: reconstruction patterns and texture patterns, and/or the projection optical device projects images of a third quantity set to the surface of the detected object based on a third exposure time, and the image acquisition device synchronously collects the images of the third quantity set on the surface of the detected object, where the types of the images of the third quantity set include: reconstruction patterns and texture patterns. It needs to be explained that in the second scanning mode, the three-dimensional scanner performs scanning based on at least two different optical conditions so as to acquire the plurality of sets of images different in brightness level. In this embodiment, the situations that the three-dimensional scanner performs scanning based on the two optical conditions and based on the three optical conditions are listed above, and the following content specifically describes processing of three sets of image sequences acquired after scanning by the three-dimensional scanner based on the three optical conditions, for example, the second scanning mode adopts the two optical conditions or other quantities of optical conditions, with image sequence processing referring to processing of the three sets of image sequences.

The texture patterns in the embodiment of the present disclosure include a red monochrome, a green monochrome and a blue monochrome, which are combined to form texture images. Of course, if the intraoral three-dimensional scanner is provided with the texture camera, the texture patterns are the texture images.

The acquiring images of a first quantity set under a first optical condition, and optionally acquiring image sequences at a first exposure time specifically includes:
the projection optical device projects the code patterns to the surface of the detected object based on the first optical condition, and the image acquisition device acquires, based on the first optical condition, code patterns modulated by the surface of the detected object, where in this embodiment, the projection optical device sequentially projects the 3 code patterns based on the first optical condition, and the camera synchronously acquires, based on the first optical condition, the 3 code patterns modulated by the surface of the detected object;
the projection optical device projects the reconstruction patterns to the surface of the detected object based on the first optical condition, and the image acquisition device acquires, based on the first optical condition, reconstruction patterns modulated by the surface of the detected object, where in this embodiment, the projection optical device sequentially projects the 3 reconstruction patterns based on the first optical condition, and the camera synchronously acquires, based on the first optical condition, the 3 reconstruction patterns (a first reconstruction sub-pattern A, a first reconstruction sub-pattern B and a first reconstruction sub-pattern C) modulated by the surface of the detected object; and
the projection optical device projects the texture patterns to the surface of the detected object based on the first optical condition, and the image acquisition device acquires, based on the first optical condition, texture patterns reflected by the surface of the detected object, where in this embodiment, the projection optical device sequentially projects the 3 texture patterns based on the first optical condition, the camera synchronously acquires, based on the first optical condition, the 3 texture patterns reflected by the surface of the detected object, and the 3 texture patterns are the red monochrome, the green monochrome and the blue monochrome respectively.

The acquiring images of a second quantity set under a second optical condition, and optionally acquiring image sequences at a second exposure time specifically includes:
the projection optical device projects the reconstruction patterns to the surface of the detected object based on the second optical condition, and the image acquisition device acquires, based on the second optical condition, reconstruction patterns modulated by the surface of the detected object, where in this embodiment, the projection optical device sequentially projects the 3 reconstruction patterns based on the second optical condition, and the image acquisition device synchronously acquires, based on the second optical condition, the 3 reconstruction patterns (a second reconstruction sub-pattern A, a second reconstruction sub-pattern B and a second reconstruction sub-pattern C) modulated by the surface of the detected object; and
the projection optical device projects the texture patterns to the surface of the detected object based on the second optical condition, and the image acquisition device acquires, based on the second optical condition, texture patterns reflected by the surface of the detected object, where in this embodiment, the projection optical device sequentially projects the 3 texture patterns based on the second optical condition, the camera synchronously acquires, based on the second optical condition, the 3 texture patterns reflected by the surface of the detected object, and the 3 texture patterns are the red monochrome, the green monochrome and the blue monochrome respectively.

The acquiring images of a third quantity set under a third optical condition, and optionally acquiring image sequences at a third exposure time specifically includes:
the projection optical device projects the reconstruction patterns to the surface of the detected object based on the third optical condition, and the image acquisition device acquires, based on the third optical condition, reconstruction patterns modulated by the surface of the detected object, where in this embodiment, the projection optical device sequentially projects the 3 reconstruction patterns based on the third optical condition, and the camera synchronously acquires, based on the third optical condition, the 3 reconstruction patterns (a third reconstruction sub-pattern A, a third reconstruction sub-pattern B and a third reconstruction sub-pattern C) modulated by the surface of the detected object; and
the projection optical device projects monochromes to the surface of the detected object based on the third optical condition, and the image acquisition device acquires, based on the third optical condition, texture patterns reflected by the surface of the detected object, where in this embodiment, the projection optical device sequentially projects the 3 texture patterns based on the third optical condition, the image acquisition device synchronously acquires, based on the third optical condition, the 3 texture patterns reflected by the surface of the detected object, and the 3 texture patterns are the red monochrome, the green monochrome and the blue monochrome respectively.

The first reconstruction sub-pattern A, the second reconstruction sub-pattern A and the third reconstruction sub-pattern A are consistent in stripe pattern, and namely, are consistent in image but only different in brightness level; the first reconstruction sub-pattern B, the second reconstruction sub-pattern B and the third reconstruction sub-pattern B are consistent in stripe pattern, and namely, are consistent in image but only different in brightness level; and the first reconstruction sub-pattern C, the second reconstruction sub-pattern C and the third reconstruction sub-pattern C are consistent in stripe pattern, and namely, are consistent in image but only different in brightness level.

The first reconstruction sub-pattern A, the second reconstruction sub-pattern A and the third reconstruction sub-pattern A are fused into a reconstruction pattern A, the first reconstruction sub-pattern B, the second reconstruction sub-pattern B and the third reconstruction sub-pattern B are fused into a reconstruction pattern B, and the first reconstruction sub-pattern C, the second reconstruction sub-pattern C and the third reconstruction sub-pattern C are fused into a reconstruction pattern C. In this embodiment, the first reconstruction sub-patterns, the second reconstruction sub-patterns and the third reconstruction sub-patterns are fused into the reconstruction patterns respectively through gray value weighted average processing, so as to remove bad data; respective stripe sequences of the first reconstruction sub-pattern A, the first reconstruction sub-pattern B and the first reconstruction sub-pattern C are determined based on the 3 code patterns, that is, respective stripe sequences of the reconstruction pattern A, the reconstruction pattern B and the reconstruction pattern C are determined, part of point clouds A on the surface of the detected object are obtained through three-dimensional reconstruction based on the reconstruction pattern A and the stripe sequence thereof, part of point clouds B on the surface of the detected object are obtained through three-dimensional reconstruction based on the reconstruction pattern B and the stripe sequence thereof, part of point clouds C on the surface of the detected object are obtained through three-dimensional reconstruction based on the reconstruction pattern C and the stripe sequence thereof, and the part of point clouds A, the part of point clouds B and the part of point clouds C constitute single dense point clouds; or three-dimensional reconstruction is respectively performed on the reconstruction sub-patterns, then point clouds obtained after three-dimensional reconstruction on the reconstruction sub-patterns are fused, and weighted average processing is specifically performed based on coordinates of the points for fusing, so as to remove the bad data.

The red monochrome acquired based on the first optical condition, the red monochrome acquired based on the second optical condition and the red monochrome acquired based on the third optical condition are fused, the green monochrome acquired based on the first optical condition, the green monochrome acquired based on the second optical condition and the green monochrome acquired based on the third optical condition are fused, and the blue monochrome acquired based on the first optical condition, the blue monochrome acquired based on the second optical condition and the blue monochrome acquired based on the third optical condition are fused, where in this embodiment, fusion of the red monochromes, the green monochromes and the blue monochromes is respectively performed through gray value weighted average processing; the texture patterns are synthesized based on the fused red monochromes, green monochromes and blue monochromes; or a first texture pattern is synthesized based on the red monochrome, the green monochrome and the blue monochrome acquired under the first optical condition, a second texture pattern is synthesized based on the red monochrome, the green monochrome and the blue monochrome acquired under the second optical condition, a third texture pattern is synthesized based on the red monochrome, the green monochrome and the blue monochrome acquired under the third optical condition, then, the texture pattern is formed by fusing the first texture pattern, the second texture pattern and the third texture pattern, and fusion of the first texture pattern, the second texture pattern and the third texture pattern is implemented through gray value weighted average processing.

A corresponding relationship between the single dense point clouds and the texture images can be determined based on a corresponding relationship between the reconstruction patterns and pixel points of the texture patterns, that is, texture information included by various points in the single dense point clouds is determined.

In the embodiment of the present disclosure, during image acquisition, the second scanning mode is adopted to perform multi-time image sequence collection, which performs exposure collection, with different exposure parameters, on the three sets of image sequences at the same position and performs fusion and three-dimensional reconstruction on the images, so that the intraoral three-dimensional scanner is applicable to acquiring three-dimensional data of to-be-detected objects with different bright-dark materials and acquiring high-quality three-dimensional data.

The code patterns in the above embodiment may be set as the reconstruction patterns for three-dimensional reconstruction, or the reconstruction patterns may serve as the code patterns, participating in coding and decoding calculation. For example, when the intraoral three-dimensional scanner works in the first scanning mode (the first scanning mode): the DLP projector (the projection optical device) sequentially projects 8 images, the monocular black and white camera (the image acquisition device) synchronously collects the 8 images relative to the DLP projector and transmits the 8 images to a computer, the computer processes the 8 images, the 5 images are stripe images, the 3 images serve as code patterns, sequences of stripes in the images are determined through setting, the 5 images may also serve as reconstruction patterns, the 3 images with dense stripes commonly serve as the reconstruction patterns, the reconstruction patterns perform three-dimensional reconstruction based on the sequences of the stripes to obtain point clouds, the other 3 images serve as the red monochrome (the texture pattern), the green monochrome (the texture pattern) and the blue monochrome (the texture pattern) respectively, and the 3 monochrome images are synthesized into a texture image, namely, texture corresponding to the point clouds.

For example, when the intraoral three-dimensional scanner works in the second scanning mode (the first scanning mode): the DLP projector sequentially projects, at the first exposure time, 8 images (3 code patterns + 3 reconstruction patterns + 3 texture patterns, where one code pattern and the reconstruction patterns are consistent and are all stripe patterns), and the image acquisition device synchronously collects the 8 images; the DLP projector sequentially projects, at the second exposure time, 6 images (3 reconstruction patterns + 3 monochromes), and the image acquisition device synchronously collects the 6 images; the DLP projector sequentially projects, at the third exposure time, 6 images (3 reconstruction patterns + 3 texture patterns), and the image acquisition device synchronously collects the 6 images; and after the 3 reconstruction patterns acquired based on the same image at different exposure times are fused, three-dimensional reconstruction is performed, and the 9 monochromes are synthesized into texture images. Of course, the 3 reconstruction patterns acquired based on the same image at different exposure times may be respectively subjected to point cloud reconstruction, and then are fused.

In the embodiment of the present disclosure, the projection sequence of the images is not limited.

S104: Image fusion and three-dimensional reconstruction are performed based on the plurality of sets of image sequences different in brightness level to generate three-dimensional data of the to-be-scanned object.

In the embodiment of the present disclosure, after the plurality of sets of image sequences different in brightness level are obtained, image fusion may be first performed, and then three-dimensional reconstruction is performed; and of course, three-dimensional reconstruction may also be first performed, and then image fusion is performed. After image fusion and three-dimensional reconstruction are finished, the three-dimensional data of the to-be-scanned is generated.

In some embodiments, the step of performing image fusion and three-dimensional reconstruction based on the plurality of sets of image sequences different in brightness level to generate three-dimensional data of the to-be-scanned object includes: fusion processing is performed on the images of the first quantity set, the images of the second quantity set and the images of the third quantity set; and three-dimensional reconstruction is performed on the fused images to generate the three-dimensional data of the to-be-scanned object, where the three-dimensional data includes point cloud data and/or texture data (i.e., the texture image).

Corresponding to the images of the quantity sets collected at the three exposure times, during image fusion and three-dimensional reconstruction, one-time three-dimensional reconstruction may be performed on the images of the quantity sets at different exposure times, and the three-dimensional data of the to-be-scanned object is directly generated by utilizing the images of the quantity sets at different exposure times.

The images collected at the three exposure times may be respectively subjected to point cloud reconstruction, and then are fused.

In the embodiment of the present disclosure, the step of performing image fusion and three-dimensional reconstruction based on the plurality of images different in brightness level to generate three-dimensional data of the to-be-scanned object further includes: point cloud reconstruction is respectively performed on the images of the first quantity set, the images of the second quantity set and the images of the third quantity set; and reconstruction results of point cloud reconstruction are fused to generate the three-dimensional data of the to-be-scanned object.

According to the invention, before the collecting a plurality of sets of image sequences different in brightness level at the same position on a to-be-scanned object, the method further includes: images on the surface of the to-be-scanned object are acquired and image uniformity is assessed; the first scanning mode is started if the images are uniform; and the second scanning mode is started if the images are not uniform, where the second scanning mode is a mode in which the plurality of sets of images different in brightness level at the same position on the to-be-scanned object are collected and then subjected to fusion and three-dimensional reconstruction. The second scanning mode may be understood as a scanning mode applicable to a high light reflection situation, in which scanning is performed on the to-be-scanned object highly reflective under different optical conditions.

In the embodiment of the present disclosure, every time when one set of image sequence is acquired, a thread may be used for setting the set of image sequence for three-dimensional reconstruction and fusion, the other thread is used for performing uniformity assessment on the image sequence, and the scanning mode for next image sequence collection is determined according to a uniformity assessment result, where uniformity assessment is determined according to whether a gray value of any image in the image sequence obviously changes or not, and preferably, is determined according to whether a gray value of a reconstruction area of any image in the image sequence obviously changes or not. If the image uniformity assessment result indicates uniformity, the first scanning mode is adopted for scanning the to-be-scanned object, and if the uniformity assessment result indicates non-uniformity, the second scanning mode is adopted to scan the to-be-scanned object.

For another option, the brightness level of the image sequence is determined according to the non-uniformity degree of the images, the brightness level adopted by the intraoral three-dimensional scanner in the second scanning mode is determined according to the non-uniformity degree of the images, that is, it is determined that the intraoral three-dimensional scanner adopts the second scanning mode A or the second scanning mode B or other modes according to the non-uniformity degree of the images. In some embodiments, in the first scanning mode, image sequences are collected based on a preset fourth optical condition, where the image sequences include code patterns, reconstruction patterns and texture patterns; and three-dimensional reconstruction is performed based on the code patterns and the reconstruction patterns to obtain point cloud images, and texture images are obtained based on the texture patterns, where the texture images correspond to the point cloud images. It is to be noted that the fourth optical condition may be identical to or different from any optical condition in the second scanning mode.

In the embodiment of the present disclosure, the first scanning mode may be understood as: the scanner collects the code patterns, the reconstruction patterns and the texture patterns according to a default optical condition, the code patterns are code stripe patterns, the reconstruction patterns are dense stripe patterns, the texture patterns are monochromes, the code patterns are used for determining the sequence of stripes in the reconstruction patterns, the reconstruction patterns perform three-dimensional reconstruction based on the sequence of the stripes to obtain point cloud data, and the three monochromes different in color are synthesized into a real color texture image. The second scanning mode may be understood as: the scanner collects code patterns, reconstruction patterns and texture patterns according to a plurality of optical conditions, and point cloud data and texture data are acquired through fusion and three-dimensional reconstruction.

FIG. 2 is a schematic diagram of an optional system for reconstructing data according to an embodiment of the present disclosure. As shown in FIG. 2, the system may include a projection optical device 21, an image acquisition device 23 and a processor 25, where
the projection optical device 21 adjusts optical conditions according to presetting, and projects image sequences different in brightness level to a to-be-scanned object, and specifically, exposure time is adjusted according to presetting, so as to adjust brightness of projected light. The projection optical device may perform light source brightness adjustment by a DLP-based digital optical processing technology, the projection optical device is the DLP projector, and the DLP projector adjusts the brightness of the projected light by adjusting the light source brightness.

The image acquisition device 23 collects a plurality of sets of image sequences different in brightness level at the same position on the to-be-scanned object. The image acquisition device includes but not limited to: the monocular black and white camera, the texture camera, a binocular black and white camera, etc.

The processor 25 respectively communicates with the projection optical device and the image acquisition device and is configured to perform image fusion and three-dimensional reconstruction based on the plurality of sets of image sequences different in brightness level to generate three-dimensional data of the to-be-scanned object.

According to the system for reconstructing data, the projection optical device 21 can adjust the exposure time according to the presetting conditions so as to adjust the brightness of the light projected to the to-be-scanned object; the image acquisition device 23 collects the plurality of sets of image sequences different in brightness level at the same position on the to-be-scanned object; and the processor 25 respectively communicates with the projection optical device and the image acquisition device and performs image fusion and three-dimensional reconstruction based on the plurality of sets of image sequences different in brightness level to generate the three-dimensional data of the to-be-scanned object. In this embodiment, if there is a local area highly bright or very dark in the scanning process, image fusion and three-dimensional reconstruction can be performed based on the plurality of sets of image sequences different in brightness level at the same position, so that the intraoral three-dimensional scanner can acquire high-quality three-dimensional data from to-be-scanned objects with different bright-dark materials, thereby solving technical problems that in the related technologies, an intraoral scanner adopts a multi-angle and multi-time scanning manner to collect intraoral tooth data, and if there is a local area highly bright or very dark in the scanned object, the camera cannot acquire uniform-brightness images.

According to another aspect of the embodiment of the present disclosure, a scanning device is further provided and includes a processor and a memory which is configured to store executable instructions of the processor. The processor is configured to execute the executable instructions to execute any above method for reconstructing data.

According to another aspect of the embodiment of the present disclosure, a computer-readable storage medium is further provided and includes stored computer programs. The computer programs, when operating, control a device where the computer-readable storage medium is located to execute any above method for reconstructing data.

The serial numbers of the above embodiments of the present disclosure are merely used for descriptions instead of representing good or bad of the embodiments.

In the above embodiments of the present disclosure, special emphasis is laid on a description of each embodiment, and for parts not described in detail in one embodiment, please refer to related descriptions in other embodiments.

It is to be understood that technical contents disclosed by the several embodiments provided by the present disclosure may be implemented by other manners. The above described embodiments of an apparatus are merely schematic, such as unit division which may be logic function division; and during practical implementation, there may be additional division manners, for example, a plurality of units or components may be combined or integrated into another system, or some characteristics may be ignored or not executed. In addition, shown or discussed mutual coupling or direct coupling or communication connection may be realized through some interfaces, and unit or module indirect coupling or communication connection may be in an electrical form or other forms.

Units described as separation parts may be or may be not physically separated, and parts for unit display may be or may be not physical units, may be located at the same position, or may be distributed on a plurality of units. Part or all of the units may be selected according to actual demands to achieve objectives of the schemes of the embodiments.

In addition, functional units in the embodiments of the present disclosure may be integrated in one processing unit, or independently and physically exist, or two or more units may be integrated in one unit. The above integrated unit may be realized in a hardware form or a form of a software functional unit.

When the integrated unit is realized in the form of the software functional unit and serves as an independent product to be sold or used, the integrated unit may be stored in the computer-readable storage medium. Based on the understanding, the technical schemes of the present disclosure essentially or parts making contribution to the prior art or all or part of the technical schemes may be embodied in a software product form. A computer software product is stored in a storage medium and includes a plurality of instructions for making a computer device (a personal computer, a server, or a network device, or the like) perform all or part of the steps of the methods in the embodiments of the present disclosure. The foregoing storage medium includes a U disk, a Read-Only Memory (ROM), a Random Access Memory (RAM), a mobile hard disk, a diskette or a light disk or other media capable of storing program code.

The above contents are merely preferred implementations of the present disclosure. It needs to be indicated that a plurality of improvements and embellishments may be made by those of ordinary skill in the art without departing from the principle of the present disclosure and should fall within the scope of protection of the present disclosure.

### Industrial applicability

The schemes provided by the embodiments of the present disclosure can be used for acquiring the three-dimensional data of the to-be-scanned object. If there is a local area highly bright or very dark in the scanning process, image fusion and three-dimensional reconstruction can be performed based on the plurality of sets of image sequences different in brightness level at the same position, so that high-quality three-dimensional data can be acquired from the to-be-scanned objects with different bright and dark materials. In the technical schemes provided by the embodiments of the present disclosure, the plurality of sets of image sequences different in brightness level at the same position on the to-be-scanned object are collected, the projection optical device projects the plurality of sets of image sequences to a surface of a detected object, and each set of image sequence includes one or more images. The projection optical device performs adjustment according to presetting, so that the images in any set of image sequence and the images in the other sets of image sequences are consistent but only different in brightness level. Through image fusion and three-dimensional reconstruction on the plurality of sets of image sequences different in brightness level at the same position, the high-quality three-dimensional data can be acquired from the to-be-scanned objects with different bright-dark materials. Thus, the technical problems that the intraoral scanner adopts the multi-angle and multi-time scanning manner to collect the intraoral tooth data, and if there is a local area highly bright or very dark in the scanned object, the camera cannot acquire the uniform-brightness images are solved.

## Claims

1. A method for reconstructing data, comprising:
acquiring images on a surface of the to-be-scanned object and assessing image uniformity; starting a first scanning mode when the images are uniform; and starting a second scanning mode when the images are not uniform, wherein the second scanning mode, comprising:
collecting a plurality of sets of image sequences, wherein the plurality of sets of image sequences are different in brightness level at the same position on a to-be-scanned object; and
performing image fusion and three-dimensional reconstruction based on the plurality of sets of image sequences to generate three-dimensional data of the to-be-scanned object.

2. The method as claimed in claim 1, wherein the collecting the plurality of sets of image sequences comprises: acquiring, by a three-dimensional scanner, the plurality of sets of image sequences at the same position based on different optical conditions.

3. The method as claimed in claim 2, further comprising:
adjusting light source brightness and/or exposure parameters of a projection optical device so as to adjust optical conditions of the three-dimensional scanner, and/or
adjusting exposure parameters and/or gain parameters of an image acquisition device so as to adjust optical conditions of the three-dimensional scanner.

4. The method as claimed in claim 3, wherein the step of acquiring, by the three-dimensional scanner, the plurality of sets of image sequences at the same position based on different optical conditions comprises:
projecting, by the projection optical device, images of a first quantity set to the surface of the detected object based on a first exposure time, synchronously collecting, by the image acquisition device, the images of the first quantity set on the surface of the detected object, wherein the types of the images of the first quantity set comprise: code patterns, reconstruction patterns and texture patterns;
projecting, by the projection optical device, images of a second quantity set to the surface of the detected object based on a second exposure time, synchronously collecting, by the image acquisition device, the images of the second quantity set on the surface of the detected object, wherein the types of the images of the second quantity set comprise: reconstruction patterns and texture patterns; and/or,
projecting, by the projection optical device, images of a third quantity set to the surface of the detected object based on a third exposure time, and synchronously collecting, by the image acquisition device, the images of the third quantity set on the surface of the detected object, wherein the types of the images of the third quantity set comprise: reconstruction patterns and texture patterns.

5. The method as claimed in claim 4, the step of performing image fusion and three-dimensional reconstruction based on the plurality of sets of image sequences to generate three-dimensional data of the to-be-scanned object comprises:
fusing reconstruction sub-patterns in the plurality of sets of image sequences into the reconstruction patterns respectively through gray value weighted average processing, wherein the reconstruction patterns comprise a first reconstruction pattern, a second reconstruction pattern, and a third reconstruction pattern;
the first quantity set comprises three code patterns, determining stripe sequences of the reconstruction patterns based on the three code patterns;
performing a three-dimensional reconstruction on a first reconstruction image and a corresponding stripe sequence to produce a first point cloud, performing a three-dimensional reconstruction on a second reconstruction image and a corresponding stripe sequence to produce a second point cloud, performing a three-dimensional reconstruction on a third reconstruction image and a corresponding stripe sequence to produce a third point cloud, determining point cloud data on the surface of the object to-be-scanned based on the first point cloud, the second point cloud, and the third point cloud.

6. The method as claimed in claim 4, wherein
fusing red monochrome acquired based on the first optical condition, red monochrome acquired based on the second optical condition and red monochrome acquired based on the third optical condition;
fusing green monochrome acquired based on the first optical condition, green monochrome acquired based on the second optical condition and green monochrome acquired based on the third optical condition;
fusing the blue monochrome acquired based on the first optical condition, the blue monochrome acquired based on the second optical condition and the blue monochrome acquired based on the third optical condition;
wherein fusion of the red monochromes, the green monochromes and the blue monochromes is respectively performed through gray value weighted average processing;
synthesizing the texture patterns based on the fused red monochromes, green monochromes and blue monochromes; or
synthesizing a first texture pattern based on the red monochrome, the green monochrome and the blue monochrome acquired under the first optical condition; synthesizing a second texture pattern based on the red monochrome, the green monochrome and the blue monochrome acquired under the second optical condition, synthesizing a third texture pattern based on the red monochrome, the green monochrome and the blue monochrome acquired under the third optical condition;
forming the texture pattern by fusing the first texture pattern, the second texture pattern and the third texture pattern, wherein fusion of the first texture pattern, the second texture pattern and the third texture pattern is implemented through gray value weighted average processing, each texture patterns comprise a red monochrome, a green monochrome, and a blue monochrome.

7. The method as claimed in claim 4, wherein the step of performing image fusion and three-dimensional reconstruction based on the plurality of sets of image sequences different in brightness level to generate three-dimensional data of the to-be-scanned object comprises:
performing fusion processing on the images of the first quantity set, the images of the second quantity set and the images of the third quantity set;
performing three-dimensional reconstruction on the fused images to generate the three-dimensional data of the to-be-scanned object, wherein the three-dimensional data comprises point cloud data and texture data; and/or,
respectively performing three-dimensional reconstruction on the images of the first quantity set, the images of the second quantity set and the images of the third quantity set; and
fusing reconstruction results of three-dimensional reconstruction to generate the three-dimensional data of the to-be-scanned object, wherein the three-dimensional data comprises point cloud data and texture data.

8. The method as claimed in claim 1, wherein before the step of collecting the plurality of sets of image sequences, the method further comprises:
determining the brightness level of the image sequences according to the non-uniformity degree of the images.

9. The method as claimed in claim 1, wherein in the first scanning mode,
image sequences are collected based on a preset fourth optical condition, wherein the image sequences comprise code patterns, reconstruction patterns and texture patterns; and
three-dimensional reconstruction is performed based on the code pattems and the reconstruction pattems to obtain point cloud images, and texture images are obtained based on the texture patterns, wherein the texture images correspond to the point cloud images.

10. The method as claimed in any of claims 1 to 9, wherein images in any set of image sequence and images in the other sets of image sequences are consistent but only different in brightness level.

11. A system for reconstructing data, comprising:
a projection optical device, configured to adjust optical conditions according to preset conditions so as to adjust light source brightness projected to a to-be-scanned object;
an image acquisition device, configured to collect a plurality of sets of image sequences, wherein the plurality of sets of image sequences are different in brightness level at the same position on the to-be-scanned object, wherein the different brightness levels are resulted from adjustment on the light source brightness by the projection optical device; and
a processor, configured to respectively communicate with the projection optical device and the image acquisition device and configured to acquire images on a surface of the to-be-scanned object and assess image uniformity; start a first scanning mode when the images are uniform; and start a second scanning mode when the images are not uniform; in the second scanning mode, perform image fusion and three-dimensional reconstruction based on the plurality of sets of image sequences different in brightness level to generate three-dimensional data of the to-be-scanned object.

12. A scanning device, comprising:
the processor of claim 11; and
a memory configured to store executable instructions of the processor,
wherein the processor is configured to cause the device of claim 11 to execute the steps of the method for reconstructing data as claimed in any one of claims 1 to 10.

13. A computer-readable storage medium, comprising stored computer programs, wherein the computer programs comprise instructions to cause the device of claim 11 to execute the steps of the method for reconstructing data as claimed in any one of claims 1 to 10.

## Patentansprüche

1. Verfahren zum Rekonstruieren von Daten, umfassend:
Erfassen von Bildern auf einer Oberfläche des abzutastenden Objekts und Bewerten von Bildeinheitlichkeit; Starten eines ersten Abtastmodus, wenn die Bilder einheitlich sind; und Starten eines zweiten Abtastmodus, wenn die Bilder nicht einheitlich sind, wobei der zweite Abtastmodus umfasst:
Sammeln einer Vielzahl von Sätzen von Bildsequenzen, wobei sich die Vielzahl von Sätzen von Bildsequenzen in der Helligkeitsstufe an derselben Position auf einem abzutastenden Objekt unterscheiden; und
Durchführen einer Bildfusion und einer dreidimensionalen Rekonstruktion basierend auf der Vielzahl von Sätzen von Bildsequenzen, um dreidimensionale Daten des abzutastenden Objekts zu erzeugen.

2. Verfahren nach Anspruch 1, wobei das Sammeln der Vielzahl von Sätzen von Bildsequenzen umfasst: Erfassen, durch einen 3D-Bildabtaster, der Vielzahl von Sätzen von Bildsequenzen an derselben Position basierend auf unterschiedlichen optischen Bedingungen.

3. Verfahren nach Anspruch 2, ferner umfassend:
Einstellen von Lichtquellenhelligkeit und/oder Belichtungsparametern einer Projektionsoptikvorrichtung, um optische Bedingungen des 3D-Bildabtasters einzustellen, und/oder
Einstellen von Belichtungsparametern und/oder Verstärkungsparametern einer Bilderfassungsvorrichtung, um optische Bedingungen des 3D-Bildabtasters einzustellen.

4. Verfahren nach Anspruch 3, wobei der Schritt des Erfassens, durch den 3D-Bildabtaster, der Vielzahl von Sätzen von Bildsequenzen an derselben Position basierend auf unterschiedlichen optischen Bedingungen umfasst:
Projizieren, durch die Projektionsoptikvorrichtung, von Bildern eines ersten Mengensatzes auf die Oberfläche des detektierten Objekts basierend auf einer ersten Belichtungszeit, synchrones Sammeln, durch die Bilderfassungsvorrichtung, der Bilder des ersten Mengensatzes auf der Oberfläche des detektierten Objekts, wobei die Arten der Bilder des ersten Mengensatzes umfassen: Codemuster, Rekonstruktionsmuster und Texturmuster;
Projizieren, durch die Projektionsoptikvorrichtung, von Bildern eines zweiten Mengensatzes auf die Oberfläche des detektierten Objekts basierend auf einer zweiten Belichtungszeit, synchrones Sammeln, durch die Bilderfassungsvorrichtung, der Bilder des zweiten Mengensatzes auf der Oberfläche des detektierten Objekts, wobei die Arten der Bilder des zweiten Mengensatzes umfassen: Rekonstruktionsmuster und Texturmuster; und/oder
Projizieren, durch die Projektionsoptikvorrichtung, von Bildern eines dritten Mengensatzes auf die Oberfläche des detektierten Objekts basierend auf einer dritten Belichtungszeit, und synchrones Sammeln, durch die Bilderfassungsvorrichtung, der Bilder des dritten Mengensatzes auf der Oberfläche des detektierten Objekts, wobei die Arten der Bilder des dritten Mengensatzes umfassen: Rekonstruktionsmuster und Texturmuster.

5. Verfahren nach Anspruch 4, wobei der Schritt des Durchführens einer Bildfusion und einer dreidimensionalen Rekonstruktion basierend auf der Vielzahl von Sätzen von Bildsequenzen zum Erzeugen dreidimensionaler Daten des abzutastenden Objekts umfasst:
Fusionieren von Rekonstruktions-Teilmustern in der Vielzahl von Sätzen von Bildsequenzen zu Rekonstruktionsmustern jeweils durch grauwertgewichtete Mittelungsverarbeitung, wobei die Rekonstruktionsmuster ein erstes Rekonstruktionsmuster, ein zweites Rekonstruktionsmuster und ein drittes Rekonstruktionsmuster umfassen;
der erste Satz umfassend drei Codemuster, und Bestimmen von Streifenfolgen der Rekonstruktionsmuster basierend auf den drei Codemustern;
Durchführen einer dreidimensionalen Rekonstruktion an einem ersten Rekonstruktionsbild und einer entsprechenden Streifenfolge, um eine erste Punktwolke zu erzeugen, Durchführen einer dreidimensionalen Rekonstruktion an einem zweiten Rekonstruktionsbild und einer entsprechenden Streifenfolge, um eine zweite Punktwolke zu erzeugen, Durchführen einer dreidimensionalen Rekonstruktion an einem dritten Rekonstruktionsbild und einer entsprechenden Streifenfolge, um eine dritte Punktwolke zu erzeugen, Bestimmen von Punktwolkendaten auf der Oberfläche des abzutastenden Objekts basierend auf der ersten Punktwolke, der zweiten Punktwolke und der dritten Punktwolke.

6. Verfahren nach Anspruch 4, wobei
das rote Monochrombild, das unter der ersten optischen Bedingung erfasst wurde, das rote Monochrombild, das unter der zweiten optischen Bedingung erfasst wurde, und das rote Monochrombild, das unter der dritten optischen Bedingung erfasst wurde, fusioniert werden;
das grüne Monochrombild, das unter der ersten optischen Bedingung erfasst wurde, das grüne Monochrombild, das unter der zweiten optischen Bedingung erfasst wurde, und das grüne Monochrombild, das unter der dritten optischen Bedingung erfasst wurde, fusioniert werden;
das blaue Monochrombild, das unter der ersten optischen Bedingung erfasst wurde, das blaue Monochrombild, das unter der zweiten optischen Bedingung erfasst wurde, und das blaue Monochrombild, das unter der dritten optischen Bedingung erfasst wurde, fusioniert werden;
wobei die Fusion der roten Monochrombilder, der grünen Monochrombilder und der blauen Monochrombilder jeweils durch grauwertgewichtete Mittelungsverarbeitung durchgeführt wird;
Synthetisieren der Texturmuster basierend auf den fusionierten roten Monochrombildern, grünen Monochrombildern und blauen Monochrombildern; oder
Synthetisieren eines ersten Texturmusters basierend auf dem roten Monochrombild, dem grünen Monochrombild und dem blauen Monochrombild, die unter der ersten optischen Bedingung erfasst wurden; Synthetisieren eines zweiten Texturmusters basierend auf dem roten Monochrombild, dem grünen Monochrombild und dem blauen Monochrombild, die unter der zweiten optischen Bedingung erfasst wurden, und Synthetisieren eines dritten Texturmusters basierend auf dem roten Monochrombild, dem grünen Monochrombild und dem blauen Monochrombild, die unter der dritten optischen Bedingung erfasst wurden;
Bilden des Texturmusters durch Fusionieren des ersten Texturmusters, des zweiten Texturmusters und des dritten Texturmusters, wobei die Fusion des ersten Texturmusters, des zweiten Texturmusters und des dritten Texturmusters durch grauwertgewichtete Mittelungsverarbeitung implementiert wird, wobei jedes Texturmuster ein rotes Monochrombild, ein grünes Monochrombild und ein blaues Monochrombild umfasst.

7. Verfahren nach Anspruch 4, wobei der Schritt des Durchführens einer Bildfusion und einer dreidimensionalen Rekonstruktion basierend auf der Vielzahl von Sätzen von Bildsequenzen, die sich in der Helligkeitsstufe unterscheiden, um dreidimensionale Daten des abzutastenden Objekts zu erzeugen, umfasst:
Durchführen einer Fusionsverarbeitung an den Bildern des ersten Satzes, den Bildern des zweiten Satzes und den Bildern des dritten Satzes;
Durchführen einer dreidimensionalen Rekonstruktion an den fusionierten Bildern, um die dreidimensionalen Daten des abzutastenden Objekts zu erzeugen, wobei die dreidimensionalen Daten Punktwolkendaten und Texturdaten umfassen; und/oder
jeweils Durchführen einer dreidimensionalen Rekonstruktion an den Bildern des ersten Satzes, den Bildern des zweiten Satzes und den Bildern des dritten Satzes und
Fusionieren von Rekonstruktionsergebnissen der dreidimensionalen Rekonstruktion, um die dreidimensionalen Daten des abzutastenden Objekts zu erzeugen, wobei die dreidimensionalen Daten Punktwolkendaten und Texturdaten umfassen.

8. Verfahren nach Anspruch 1, wobei vor dem Schritt des Sammelns der Vielzahl von Sätzen von Bildsequenzen das Verfahren ferner umfasst:
Bestimmen der Helligkeitsstufe der Bildsequenzen gemäß dem Grad der Uneinheitlichkeit der Bilder.

9. Verfahren nach Anspruch 1, wobei in dem ersten Abtastmodus
Bildsequenzen basierend auf einer vorgegebenen vierten optischen Bedingung gesammelt werden, wobei die Bildsequenzen Codemuster, Rekonstruktionsmuster und Texturmuster umfassen; und
eine dreidimensionale Rekonstruktion basierend auf den Codemustern und den Rekonstruktionsmustern durchgeführt wird, um Punktwolkenbilder zu erhalten, und Texturbilder basierend auf den Texturmustern erhalten werden, wobei die Texturbilder den Punktwolkenbildern entsprechen.

10. Verfahren nach einem der Ansprüche 1 bis 9, wobei die Bilder in einem beliebigen Satz von Bildsequenzen und die Bilder in den anderen Sätzen von Bildsequenzen konsistent sind und sich lediglich in der Helligkeitsstufe unterscheiden.

11. System zum Rekonstruieren von Daten, umfassend:
eine Projektionsoptikvorrichtung, die konfiguriert ist, um optische Bedingungen gemäß vorgegebenen Bedingungen einzustellen, um eine Lichtquellenhelligkeit einzustellen, die auf ein abzutastendes Objekt projiziert wird;
eine Bilderfassungsvorrichtung, die konfiguriert ist, um eine Vielzahl von Sätzen von Bildsequenzen zu sammeln, wobei sich die Vielzahl von Sätzen von Bildsequenzen in der Helligkeitsstufe an derselben Position auf dem abzutastenden Objekt unterscheiden, wobei die unterschiedlichen Helligkeitsstufen aus der Einstellung der Lichtquellenhelligkeit durch die Projektionsoptikvorrichtung resultieren; und
einen Prozessor, der konfiguriert ist, um jeweils mit der Projektionsoptikvorrichtung und der Bilderfassungsvorrichtung zu kommunizieren, und konfiguriert ist, um Bilder auf einer Oberfläche des abzutastenden Objekts zu erfassen und eine Bildeinheitlichkeit zu bewerten; einen ersten Abtastmodus zu starten, wenn die Bilder einheitlich sind; und einen zweiten Abtastmodus zu starten, wenn die Bilder nicht einheitlich sind; im zweiten Abtastmodus eine Bildfusion und eine dreidimensionale Rekonstruktion basierend auf der Vielzahl von Sätzen von Bildsequenzen durchzuführen, die sich in der Helligkeitsstufe unterscheiden, um dreidimensionale Daten des abzutastenden Objekts zu erzeugen.

12. Abtastvorrichtung, umfassend:
den Prozessor nach Anspruch 11 und
einen Speicher, der konfiguriert ist, um ausführbare Anweisungen des Prozessors zu speichern,
wobei der Prozessor konfiguriert ist, um die Vorrichtung nach Anspruch 11 zu veranlassen, die Schritte des Verfahrens zum Rekonstruieren von Daten nach einem der Ansprüche 1 bis 10 auszuführen.

13. Computerlesbares Speichermedium, umfassend gespeicherte Computerprogramme, wobei die Computerprogramme Anweisungen umfassen, um die Vorrichtung nach Anspruch 11 zu veranlassen, die Schritte des Verfahrens zum Rekonstruieren von Daten nach einem der Ansprüche 1 bis 10 auszuführen.

## Revendications

1. Procédé permettant de reconstruire des données, comprenant :
l'acquisition d'images sur une surface de l'objet à balayer et l'évaluation d'une uniformité d'image ; le démarrage d'un premier mode de balayage lorsque les images sont uniformes ; et le démarrage d'un second mode de balayage lorsque les images ne sont pas uniformes, dans lequel le second mode de balayage, comprenant :
la collecte d'une pluralité d'ensembles de séquences d'images, dans lequel la pluralité d'ensembles de séquences d'images sont différents en niveau de luminosité au niveau de la même position sur un objet à balayer ; et
la réalisation d'une fusion d'images et d'une reconstruction tridimensionnelle sur la base de la pluralité d'ensembles de séquences d'images pour générer des données tridimensionnelles de l'objet à balayer.

2. Procédé selon la revendication 1, dans lequel le rassemblement de la pluralité d'ensembles de séquences d'images comprend : l'acquisition, par un dispositif de balayage tridimensionnel, de la pluralité d'ensembles de séquences d'images au niveau de la même position sur la base de conditions optiques différentes.

3. Procédé selon la revendication 2, comprenant en outre :
l'ajustement d'une luminosité de source de lumière et/ou de paramètres d'exposition d'un dispositif optique de projection de manière à ajuster des conditions optiques du dispositif de balayage tridimensionnel, et/ou
l'ajustement de paramètres d'exposition et/ou de paramètres de gain d'un dispositif d'acquisition d'images de manière à ajuster des conditions optiques du dispositif de balayage tridimensionnel.

4. Procédé selon la revendication 3, dans lequel l'étape consistant à acquérir, par le dispositif de balayage tridimensionnel, la pluralité d'ensembles de séquences d'images au niveau de la même position sur la base de conditions optiques différentes comprend :
la projection, par le dispositif optique de projection, d'images d'une première quantité définie sur la surface de l'objet détecté sur la base d'un premier moment d'exposition, la collecte synchronisée, par le dispositif d'acquisition d'images, des images de la première quantité définie sur la surface de l'objet détecté, dans lequel les types des images de la première quantité définie comprennent : des modèles de code, des modèles de reconstruction et des modèles de texture ;
la projection, par le dispositif optique de projection, d'images d'une deuxième quantité définie sur la surface de l'objet détecté sur la base d'un deuxième moment d'exposition, la collecte synchronisée, par le dispositif d'acquisition d'images, des images de la deuxième quantité définie sur la surface de l'objet détecté, dans lequel les types des images de la deuxième quantité définie comprennent : des modèles de reconstruction et des modèles de texture ; et/ou,
la projection, par le dispositif optique de projection, d'images d'une troisième quantité définie sur la surface de l'objet détecté sur la base d'un troisième moment d'exposition, et la collecte synchronisée, par le dispositif d'acquisition d'images, des images de la troisième quantité définie sur la surface de l'objet détecté, dans lequel les types des images de la troisième quantité définie comprennent : des modèles de reconstruction et des modèles de texture.

5. Procédé selon la revendication 4, l'étape consistant à réaliser une fusion d'images et une reconstruction tridimensionnelle sur la base de la pluralité d'ensembles de séquences d'images pour générer des données tridimensionnelles de l'objet à balayer comprend :
la fusion de sous-modèles de reconstruction dans la pluralité d'ensembles de séquences d'images dans les modèles de reconstruction respectivement par le biais d'un traitement par moyenne pondérée des niveaux de gris, dans lequel les modèles de reconstruction comprennent un premier modèle de reconstruction, un deuxième modèle de reconstruction, et un troisième modèle de reconstruction ;
la première quantité définie comprenant trois modèles de code, et déterminant des séquences de bandes des modèles de reconstruction sur la base des trois modèles de code ;
la réalisation d'une reconstruction tridimensionnelle sur une première image de reconstruction et une séquence de bandes correspondante pour produire un premier nuage de points, la réalisation d'une reconstruction tridimensionnelle sur une deuxième image de reconstruction et une séquence de bandes correspondante pour produire un deuxième nuage de points, la réalisation d'une reconstruction tridimensionnelle sur une troisième image de reconstruction et une séquence de bandes correspondante pour produire un troisième nuage de points, la détermination de données de nuage de points sur la surface de l'objet à balayer sur la base du premier nuage de points, du deuxième nuage de points et du troisième nuage de points.

6. Procédé selon la revendication 4, dans lequel
la fusion d'un monochrome rouge acquis sur la base de la première condition optique, d'un monochrome rouge acquis sur la base de la deuxième condition optique et d'un monochrome rouge acquis sur la base de la troisième condition optique ;
la fusion d'un monochrome vert acquis sur la base de la première condition optique, d'un monochrome vert acquis sur la base de la deuxième condition optique et d'un monochrome vert acquis sur la base de la troisième condition optique ;
la fusion du monochrome bleu acquis sur la base de la première condition optique, du monochrome bleu acquis sur la base de la deuxième condition optique et du monochrome bleu acquis sur la base de la troisième condition optique ;
dans lequel la fusion des monochromes rouges, des monochromes verts et des monochromes bleus est respectivement réalisée par le biais d'un traitement par moyenne pondérée des niveaux de gris ;
la synthèse des modèles de texture sur la base des monochromes rouges, des monochromes verts et des monochromes bleus fusionnés ; ou
la synthèse d'un premier modèle de texture sur la base du monochrome rouge, du monochrome vert et du monochrome bleu acquis dans la première condition optique ; la synthèse d'un deuxième modèle de texture sur la base du monochrome rouge, du monochrome vert et du monochrome bleu acquis dans la deuxième condition optique, la synthèse d'un troisième modèle de texture sur la base du monochrome rouge, du monochrome vert et du monochrome bleu acquis dans la troisième condition optique ;
la formation du modèle de texture en fusionnant le premier modèle de texture, le deuxième modèle de texture et le troisième modèle de texture, dans lequel une fusion du premier modèle de texture, du deuxième modèle de texture et du troisième modèle de texture est mise en œuvre par le biais d'un traitement par moyenne pondérée des niveaux de gris, chaque modèle de texture comprenant un monochrome rouge, un monochrome vert, et un monochrome bleu.

7. Procédé selon la revendication 4, dans lequel l'étape consistant à réaliser une fusion d'images et une reconstruction tridimensionnelle sur la base de la pluralité d'ensembles de séquences d'images de niveau de luminosité différent pour générer des données tridimensionnelles de l'objet à balayer comprend :
la réalisation d'un traitement de fusion sur les images de la première quantité définie, les images de la deuxième quantité définie et les images de la troisième quantité définie ;
la réalisation d'une reconstruction tridimensionnelle sur les images fusionnées pour générer les données tridimensionnelles de l'objet à balayer, dans lequel les données tridimensionnelles comprennent des données de nuage de points et des données de texture ; et/ou,
la réalisation respective d'une reconstruction tridimensionnelle sur les images de la première quantité définie, les images de la deuxième quantité définie et les images de la troisième quantité définie ; et
la fusion de résultats de reconstruction de la reconstruction tridimensionnelle pour générer les données tridimensionnelles de l'objet à balayer, dans lequel les données tridimensionnelles comprennent des données de nuage de points et des données de texture.

8. Procédé selon la revendication 1, dans lequel, avant l'étape consistant à collecter la pluralité d'ensembles de séquences d'images, le procédé comprend en outre :
la détermination du niveau de luminosité des séquences d'images selon le degré de non-uniformité des images.

9. Procédé selon la revendication 1, dans lequel, dans le premier mode de balayage,
des séquences d'images sont collectées sur la base d'une quatrième condition optique prédéfinie, dans lequel les séquences d'images comprennent des modèles de code, des modèles de reconstruction et des modèles de texture ; et
une reconstruction tridimensionnelle est réalisée sur la base des modèles de code et des modèles de reconstruction pour obtenir des images de nuage de points, et des images de texture sont obtenues sur la base des modèles de texture, dans lequel les images de texture correspondent aux images de nuage de points.

10. Procédé selon l'une quelconque des revendications 1 à 9, dans lequel des images dans un quelconque ensemble de séquences d'images et des images dans les autres ensembles de séquences d'images sont cohérentes mais seulement différentes en termes de niveau de luminosité.

11. Système permettant de reconstruire des données, comprenant :
un dispositif optique de projection, configuré pour ajuster des conditions optiques selon des conditions prédéfinies de manière à ajuster une luminosité de source de lumière projetée sur un objet à balayer ;
un dispositif d'acquisition d'images, configuré pour collecter une pluralité d'ensembles de séquences d'images, dans lequel la pluralité d'ensembles de séquences d'images ont un niveau de luminosité différent au niveau de la même position sur l'objet à balayer, dans lequel les différents niveaux de luminosité sont le résultat d'un ajustement de la luminosité de source de lumière par le dispositif optique de projection ; et
un processeur, configuré pour communiquer respectivement avec le dispositif optique de projection et le dispositif d'acquisition d'images, et configuré pour acquérir des images sur une surface de l'objet à balayer et évaluer une uniformité d'image ; démarrer un premier mode de balayage lorsque les images sont uniformes ; et démarrer un second mode de balayage lorsque les images ne sont pas uniformes ; dans le second mode de balayage, réaliser une fusion d'images et une reconstruction tridimensionnelle sur la base de la pluralité d'ensembles de séquences d'images de niveau de luminosité différent afin de générer des données tridimensionnelles de l'objet à balayer.

12. Dispositif de balayage, comprenant :
le processeur selon la revendication 11 ; et
une mémoire configurée pour stocker des instructions exécutables du processeur,
dans lequel le processeur est configuré pour amener le dispositif selon la revendication 11 à exécuter les étapes du procédé permettant de reconstruire des données selon l'une quelconque des revendications 1 à 10.

13. Support de stockage lisible par un ordinateur, comprenant des programmes informatiques stockés, dans lequel les programmes informatiques comprennent des instructions pour amener le dispositif selon la revendication 11 à exécuter les étapes du procédé permettant de reconstruire des données selon l'une quelconque des revendications 1 à 10.
